(19) Europäisches Patentamt

European Patent Office

Office européen des brevets

(11) **EP 1 683 481 A1**

(12) **EUROPEAN PATENT APPLICATION**

(43) Date of publication:
**26.07.2006 Bulletin 2006/30**

(51) Int Cl.:
***A61B 5/053*** (2006.01)

(21) Application number: **06001348.9**

(22) Date of filing: **23.01.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **24.01.2005 JP 2005015871**

(71) Applicant: **TANITA CORPORATION**
**Tokyo 174-0063 (JP)**

(72) Inventors:
 • **Takehara, Katsumi**
  **Itabashi-ku**
  **Tokyo 174-0063 (JP)**
 • **Fukuda, Yoshinori**
  **Itabashi-ku**
  **Tokyo 174-0063 (JP)**

(74) Representative: **Müller-Boré & Partner**
**Patentanwälte**
**Grafinger Strasse 2**
**81671 München (DE)**

(54) **Bioelectrical impedance measuring device and body composition measuring apparatus**

(57)    There are provided a bioelectrical impedance measuring device which calculates bioelectrical impedance parameter values by use of an AD converter incorporated in a low-cost general-purpose microcontroller and a body composition measuring apparatus using the device. The bioelectrical impedance measuring device measures voltages generated in a living body according to alternating currents of predetermined frequencies applied to the living body and comprises digital data acquiring means for acquiring digital data by sampling the measurement signals of the voltages by sampling frequencies which are not higher than the Nyquist frequencies and calculation means for calculating bioelectrical impedance parameter values based on the digital data. Thus, since high-speed processing is not needed at the time of conversion to the digital data, sampling can be processed by the AD converter in the low-cost general-purpose microcontroller, thereby making cost reduction possible.

FIG. 1

EP 1 683 481 A1

**Description**

BACKGROUND OF THE INVENTION

(i) Field of the Invention

The present invention relates to a bioelectrical impedance measuring device and a body composition measuring apparatus using the device.

(ii) Description of the Related Art

A conventional low-cost body composition measuring apparatus using a general-purpose microcontroller estimates body compositions by sole use of the absolute value of a bioelectrical impedance based on a voltage generated according to an alternating current applied to a living body. However, it has been understood from studies in recent years that in addition to the absolute value of the bioelectrical impedance, the parameter values of the bioelectrical impedance such as the phase difference of the bioelectrical impedance and the resistance component value and reactance component value of the bioelectrical impedance that are determined from the above absolute value and phase difference are also useful for estimation of body compositions.

Next, the principle of calculations of parameters based on conventional bioelectrical impedance measurement will be described briefly. First of all, the parameter values of bioelectrical impedance are in the following relationships.

Absolute Value of Bioelectrical Impedance: $|Z| = (R^2 + X^2)^{1/2}$

Phase Difference between Applied Current and Measured Voltage: $\phi = \tan^{-1}(X/R)$

Resistance Component (hereinafter referred to as "resistance value") of Bioelectrical Impedance: $R = |Z|\cos()$

Reactance Component (hereinafter referred to as "reactance value") of Bioelectrical Impedance: $X = |Z|\sin(\phi)$

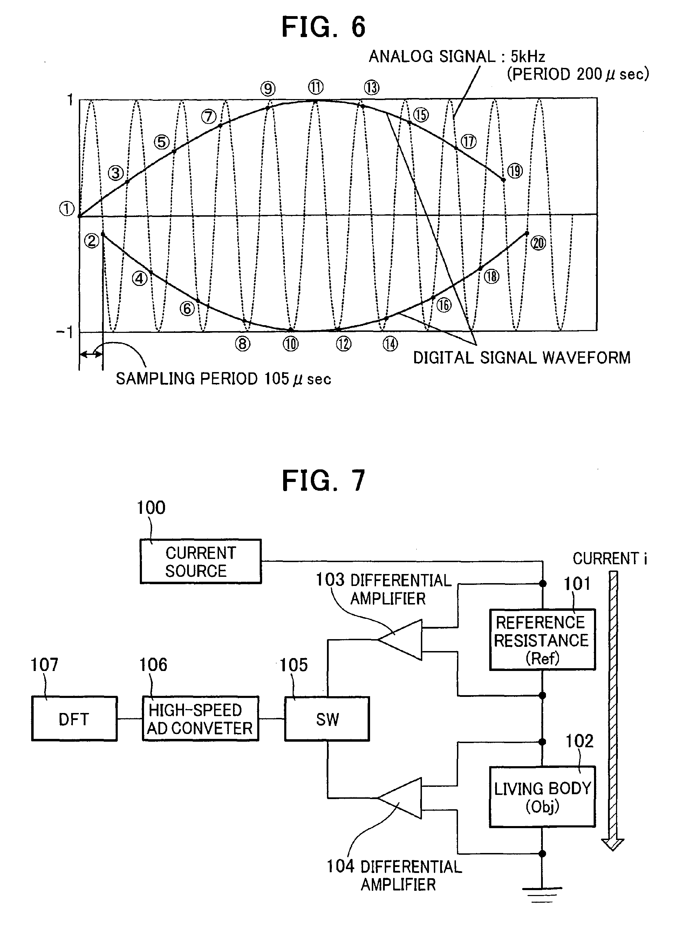

Next, a known bioelectrical impedance parameter calculation model shown in Fig. 7 will be described. In this model, a current source 100 that produces a bioelectrical impedance measuring current i is connected to a reference resistance (Ref) 101 whose resistance value is known and a living body (Obj) 102 to apply the current i thereto. The reference resistance (Ref) 101 and living body (Obj) 102 are connected to differential amplifiers 103 and 104 that receive potential differences that occur in the reference resistance (Ref) 101 and living body (Obj) 102 upon application of the current i as analog signals $A_{Ref}$ and $A_{obj}$, respectively. The differential amplifiers 103 and 104 are connected to a high-speed AD converter 106 which converts the analog signals $A_{Ref}$ and $A_{obj}$ into corresponding digital signals $D_{Ref}$ and $D_{obj}$ via an SW 105 which switches connection to either of the differential amplifiers 103 and 104. The high-speed AD converter 106 is connected to an impedance parameter calculation section 107 which includes the DFT (Discrete Fourier Transform) process that determines amplitude and phase spectra based on the digital signals $D_{Ref}$ and $D_{obj}$.

The high-speed AD converter 106 is an AD converter which is capable of high-speed processing of sampling in conversion of analog signals to corresponding digital signals. That is, the converter conducts sampling by a sampling frequency not lower than the Nyquist frequency (frequency that is twice the frequency of measurement signal) and samples about 20 to 30 points in one period of the waveform of the analog signal for the sake of accuracy since the measurement object is a living body. Further, at that time, sampling is started from the same phase of the above current i to be applied, and the period of the analog signal to be sampled is an integer period.

Next, the above DFT process in the impedance parameter calculation section 107 will be described. First, Fourier transform is a process of resolving a digital signal resulting from sampling an analog signal along with the time axis into a sinusoidal component contained in the digital signal. It calculates the spectra of the amplitude and phase of the sinusoidal component.

In the above model, as described below, the spectra of the amplitude and phase of sinusoidal component obtained by conducting the above Fourier transform on the above digital signals $D_{Ref}$ and $D_{Obj}$ are calculated, and the parameters of bioelectrical impedance are calculated by use of the above spectra based on known formulas for calculating the parameters.

First, the DFT process is conducted on the above digital signals $D_{Ref}$ and $D_{Obj}$ by the following formula and is represented by a complex Fourier spectrum $S_k$ which is formed by the real part and the imaginary part. That is,

$$S_k = \Sigma[D(n) \times \cos\{(2\pi kn)/N\}] - j \times \Sigma[D(n) \times \sin\{(2\pi kn)/N\}]$$

In the above formula, n represents a sampling number, N represents the total number of samples, k represents a spectrum number, and D(n) represents the $n^{th}$ sampling data. Further, the value of the spectrum number k is the same as the integer value of the integer period of the analog signal to be sampled.

Further, the complex Fourier spectrum $S_k$ is represented by the following formula wherein $Real_k$ represents the above real part and $Img_k$ represents the above imaginary part.

$$S_k = Real_k + Img_k$$

Therefore, with respect to the above digital signals $D_{Ref}$ and $D_{Obj}$, the above complex Fourier spectra are represented by the following formulas.

$$S_{Ref} = Real_{Ref} + jImg_{Ref}$$

$$S_{Obj} = Real_{Obj} + jImg_{Obj}$$

Further, the above amplitude spectra are represented by the ) following formulas by the absolute values of the above complex Fourier spectra $S_{Ref}$ and $S_{Obj}$.

$$|S_{Ref}| = \{(Real_{Ref})^2 + (Img_{Ref})^2\}^{1/2}$$

$$|S_{Obj}| = \{(Real_{Obj})^2 + (Img_{Obj})^2\}^{1/2}$$

Further, the above phase spectra $\theta_{Ref}$ and $\theta_{Obj}$ are represented by the following formulas.

$$\theta_{Ref} = \tan^{-1}(Img_{Ref}/Real_{Ref})$$

$$\theta_{Obj} = \tan^{-1}(Img_{Obj}/Real_{Obj})$$

Therefore, the absolute value $|Z_{Obj}|$ of bioelectrical impedance is determined by the following formula based on the ratio of the above amplitude spectra, because the currents i which pass through the above reference resistance (Ref) 101 and the above living body (Obj) 102 are the same and the impedance of the reference resistance (Ref) 101 is known.

$$|Z_{Obj}| = |Z_{Ref}| \times |S_{Obj}|/|S_{Ref}|$$

Further, the phase difference $\phi$ between the applied current and the measured voltage is determined from the following formula based on the above phase spectra.

$$\phi = \theta_{Obj} - \theta_{Obj}$$

Further, the resistance component R and reactance component X of the bioelectrical impedance are determined by the following formulas based on the above absolute value $|Z_{Obj}|$ of the bioelectrical impedance and the phase difference $\phi$.

$$R = |Z_{Obj}|\cos(\phi)$$

$$X = |Z_{Obj}|\sin(\phi)$$

There is disclosed a body composition measuring apparatus which makes more detailed estimations of body compositions by use of the above bioelectrical impedance parameter values determined as described above (for example, refer to Patent Literature 1).

Patent Literature 1

Japanese Patent Laid-Open Publication No. 255120/2004

However, the above body composition measuring apparatus which makes more detailed estimations of body compositions by use of the above bioelectrical impedance parameter values require an IC and complex analog circuit which are exclusively used for calculations of the above parameter values. Particularly, as an AD converter which digitizes an analog voltage signal measured for calculating a bioelectrical impedance, a high-speed AD converter is required to process sampling data obtained by sampling the signal by a sampling frequency which is not lower than the Nyquist frequency so as to improve measurement accuracy, thereby causing an increase in costs.

Thus, an object of the present invention is to solve the above problem and provide a bioelectrical impedance measuring device which calculates bioelectrical impedance parameter values by use of an AD converter incorporated in a low-cost general-purpose microcontroller and a body composition measuring apparatus using the device.

SUMMARY OF THE INVENTION

To solve the above problem, the present invention provides a bioelectrical impedance measuring device which measures voltages generated in a living body according to alternating currents of predetermined frequencies applied to the living body, the device comprising:

digital data acquiring means, and
calculation means,
wherein
the digital data acquiring means acquires digital data by sampling the measurement signals of the voltages by sampling frequencies that are not higher than the Nyquist frequencies, and
the calculation means calculates bioelectrical impedance parameter values based on the digital data.

Further, the digital data acquiring means samples the number of samples required to digitize one period of the measurement signal by such a sampling frequency that acquires the number over a number of periods of the measurement signal.

Further, the digital data acquiring means takes an integer period as the sampling period.

Further, the digital data acquiring means comprises shaping means for shaping a waveform formed by the sampling when a number of samplings are conducted on one period of the measurement signal.

Further, the digital data acquiring means comprises sampling frequency switching means for switching the sampling frequency automatically according to the frequency of the measurement signal.

Further, the calculation means calculates the parameter values by the DFT (Discrete Fourier Transform) process based on the digital data.

The present invention also provides a body composition measuring apparatus comprising:

the above bioelectrical impedance measuring device, and
body composition calculating means for calculating indicators
associated with body compositions such as body fat, muscles, body water and bones based on the acquired parameter values.

The bioelectrical impedance measuring device of the present invention measures voltages generated in a living body according to alternating currents of predetermined frequencies applied to the living body and comprises digital data acquiring means for acquiring digital data by sampling the measurement signals of the voltages by sampling frequencies that are not higher than the Nyquist frequencies and calculation means for calculating bioelectrical impedance parameter values based on the digital data. Further, the digital data acquiring means samples the number of samples required to digitize one period of the measurement signal by such a sampling frequency that acquires the number over a number of periods of the measurement signal. Thus, since high-speed processing is not needed at the time of conversion to the digital data, sampling can be processed by a low-cost AD converter incorporated in a general-purpose microcontroller, thereby making cost reduction possible.

Further, the digital data acquiring means takes an integer period as the sampling period. Further, the digital data acquiring means comprises shaping means for shaping a waveform formed by the sampling when a number of samplings are conducted on one period of the measurement signal. Thus, smoothly continuous data suitable for the above DFT process for calculating bioelectrical impedance parameter values based on sampling data are obtained. This can prevent errors that occur when discontinuous data exist in the DFT process which is carried out on the premise that sampling data continue indefinitely.

Further, the digital data acquiring means comprises sampling frequency switching means for switching the sampling frequency automatically according to the frequency of the measurement signal. Thereby, no cumbersome operations are needed, and the present device can deal with measurement of bioelectrical impedance by a number of frequencies.

Further, the calculation means calculates the parameter values by the DFT process based on the digital data. Thereby, the present device can calculate the above bioelectrical impedance parameters more easily than the FFT (Fast Fourier Transform) process which performs Fourier transform at high speed.

Further, the body composition measuring apparatus of the present invention comprises the above bioelectrical impedance measuring device and body composition calculating means for calculating indicators associated with body compositions such as body fat, muscles, body water and bones based on the acquired parameter values. Thus, since the above bioelectrical impedance measuring device can highly accurately measure a bioelectrical impedance by a low frequency in particular, a bioelectrical impedance measured by 50 kHz has high correlations with body compositions such as a total water content, body fat mass, body fat percentage, basal metabolism and bone mass, and a bioelectrical impedance measured by 6.25 kHz has a high correlation with an extracellular fluid volume. Further, since an intracellular fluid volume resulting from subtracting the extracellular fluid volume from the total water content has a high correlation with a muscle amount, highly reliable data can be obtained in calculations of body compositions.

BRIEF DESCRIPTION OF THE DRAWINGS

Fig. 1 is a block diagram illustrating the constitution of a body composition measuring apparatus of the present example.
Fig. 2 is a flowchart illustrating the operation of the main routine of the body composition measuring apparatus of the present example.
Fig. 3 is a flowchart illustrating the operation of a bioelectrical impedance measurement subroutine of Example 1.
Fig. 4 is a diagram illustrating an example of undersampling at a measurement frequency of 50 kHz.
Fig. 5 is a flowchart illustrating the operation of a bioelectrical impedance measurement subroutine of Example 2.
Fig. 6 is a diagram illustrating an example of undersampling at a measurement frequency of 5 kHz.
Fig. 7 is a model diagram for illustrating the principle of calculations of the parameters of bioelectrical impedance.

DETAILED DESCRIPTION OF THE PREFERRED EMBODIMENTS

The bioelectrical impedance measuring device of the present invention is a bioelectrical impedance measuring device which measures voltages generated in a living body according to alternating currents of predetermined frequencies applied to the living body,
the device comprising:

digital data acquiring means, and
calculation means,
wherein
the digital data acquiring means acquires digital data by sampling the measurement signals of the voltages by sampling frequencies that are not higher than the Nyquist frequencies, and
the calculation means calculates bioelectrical impedance parameter values based on the digital data.

Further, the digital data acquiring means samples the number of samples required to digitize one period of the measurement signal by such a sampling frequency that acquires the number over a number of periods of the measurement signal.

Further, the digital data acquiring means takes an integer period as the sampling period.

Further, the digital data acquiring means comprises shaping means for shaping a waveform formed by the sampling when a number of samplings are conducted on one period of the measurement signal.

Further, the digital data acquiring means comprises sampling frequency switching means for switching the sampling frequency automatically according to the frequency of the measurement signal.

Further, the calculation means calculates the parameter values by the DFT process based on the digital data.

The body composition measuring apparatus of present invention comprises the above bioelectrical impedance measuring device and body composition calculating means for calculating indicators associated with body compositions such as body fat, muscles, body water and bones based on the acquired parameter values.

Example 1

Example 1 of the present invention will be described by use of a body composition measuring apparatus comprising a bioelectrical impedance measuring device using undersampling of sampling one point per period of analog signal waveform as an example.

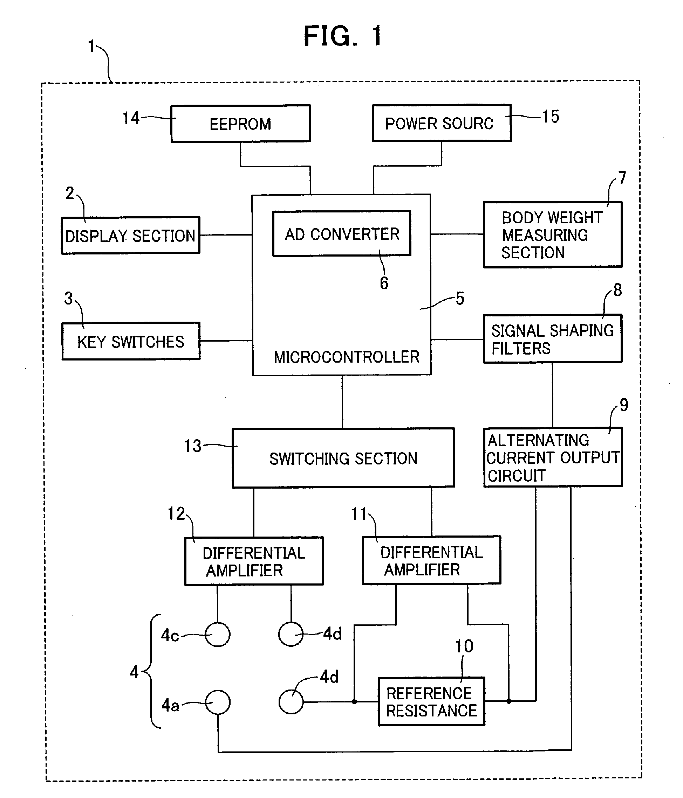

Firstly, the constitution of the body composition measuring apparatus of the present invention will be described by use of Fig. 1. A body composition measuring apparatus 1 comprises a display section 2, key switches 3, and a bioelectrical impedance measuring electrodes 4 that comprise current applying electrodes 4a and 4b and voltage measuring electrodes 4c and 4d. The display section 2 displays measurement results and guidance. The key switches 3 are used for various settings and input operations. The current applying electrodes 4a and 4b apply a predetermined current to a living body. The voltage measuring electrodes 4c and 4d measure a potential difference between body parts.

The display section 2 and the key switches 3 are connected to a microcontroller 5 which controls the body composition measuring apparatus 1 and performs calculations. The microcontroller 5 is a general-purpose microcontroller incorporating an AD converter 6 that has low-speed processing power. Further, the microcontroller 5 is connected to a body weight measuring section 7 which measures a body weight. In addition, the microcontroller 5 is also connected to an alternating current output circuit 9 which outputs an alternating current via a signal shaping filter 8 which shapes a rectangular wave output from the microcontroller 5 to measure a bioelectrical impedance into a sinusoidal signal of desired frequency. The alternating current output circuit 9 is connected to the current applying electrodes 4a and 4b. It is connected to the current applying electrode 4b via a reference resistance 10.

A differential amplifier 11 which acquires a potential difference that occurs in the reference resistance 10 based on an applied current and a differential amplifier 12 which acquires a potential difference between the voltage measuring electrodes 4c and 4d to acquire a potential difference that occurs in a living body are connected to the microcontroller 5 via a switching section 13 which switches between potential difference signals from the differential amplifiers 11 and 12. Further, an EEPROM 14 which stores measured data temporarily and a power source 15 which supplies electric power to the body composition measuring apparatus 1 are connected to the microcontroller 5.

In addition to the AD converter 6, the microcontroller 5 further comprises a control section which controls the body composition measuring apparatus 1, an arithmetic section which calculates bioelectrical impedance parameters and indicators associated with body compositions, a rectangular wave output section which outputs a rectangular wave so as to measure a bioelectrical impedance, a storage section which stores various data and preset calculation formulas, and a sampling period setting section which sets the sampling period of the above AD converter according to measurement frequency.

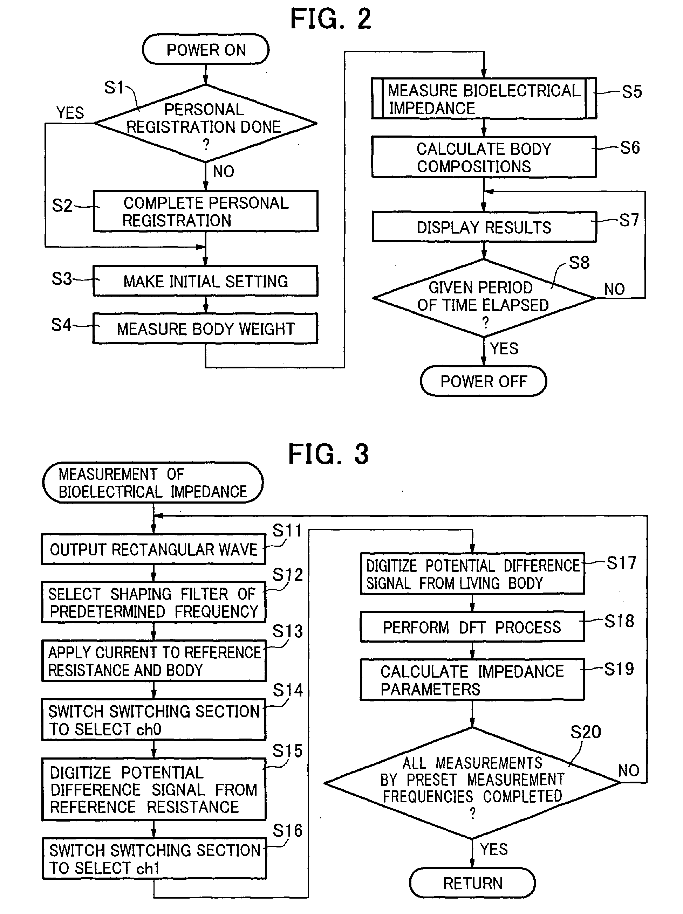

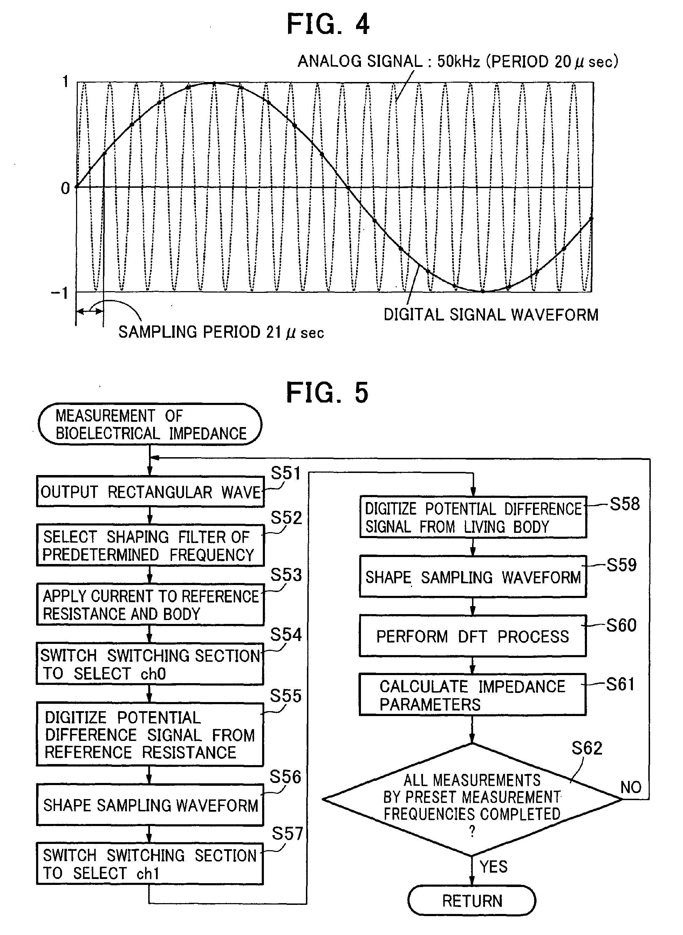

Next, the operation of the body composition measuring apparatus 1 will be described by use of Figs. 2 to 4. Fig. 2 is a flowchart illustrating the operation of the main routine, Fig. 3 is a flowchart illustrating the operation of a bioelectrical impedance measurement subroutine, and Fig. 4 is a graph illustrating the results of sampling a measurement frequency of 50 kHz.

First, when the body composition measuring apparatus 1 is turned on in Fig. 2, it is determined in STEP S1 whether personal data for calculating body compositions such as age, gender and a body height are already stored in the storage section in the microcontroller 5 to complete personal registration. If the personal registration has been done, initial setting of the body composition measuring apparatus 1 is made in STEP S3. If the personal registration has not been done, a subject enters personal data in STEP S2 by use of the key switches 3 in accordance with guidance displayed in the display section 2 by the microcontroller 5 to urge the subject to complete the personal registration, and then the above STEP S3 is carried out. After completion of the initial setting in the above STEP S3, the body weight of the subject is measured by the body weight measuring section 7 in STEP S4, and measurement of bioelectrical impedance to be described later is carried out in STEP S5 by use of the flowchart of Fig. 3. In STEP S6, indicators associated with body compositions such as body fat, body water, muscles and bones are calculated based on the above personal data and the above measured body weight and bioelectrical impedance in the arithmetic section of the microcontroller 5, and the measurement results are displayed in the display section 2 in STEP S7. In STEP S8, it is determined whether the above data have been displayed for a given period of time, and if the data have not been displayed for the given period of time, the data remain displayed, while if the data have been displayed for the given period of time, the body composition measuring apparatus 1 is tuned off automatically, thereby ending the measurement.

Next, the above measurement of the bioelectrical impedance will be described in accordance with the flowchart of Fig. 3. In the measurement of the bioelectrical impedance, a rectangular wave is output from the rectangular wave output section in the microcontroller 5 in STEP S11, and the signal shaping filter 8 is controlled by the control section in the microcontroller 5 to shape the above rectangular wave into a waveform of frequency desired as measurement frequency to measure a bioelectrical impedance, and an appropriate filter is selected to shape the rectangular wave into a sinusoidal waveform.

In this case, the bioelectrical impedance is measured by two measurement frequencies of 50 kHz and 6.25 kHz, and two filters which shape the rectangular wave into 50 kHz and 6.25 kHz, respectively, are prepared as the above signal shaping filter.

For example, when the filter of 50 kHz is selected by the control section in the microcontroller 5 in the above STEP S12, an alternating current of 50 kHz is output from the alternating current output circuit 9 based on the above shaped sinusoidal signal and applied to the reference resistance 10 and between the current applying electrodes 4a and 4b. In STEP S14, the switching section 13 is controlled by the control section in the microcontroller 5 and switched to the differential amplifier 11 side (referred to as "ch0") so as to acquire a potential difference that occurs in the reference resistance 10.

In STEP S15, the above acquired potential difference as an analog voltage signal is converted into digital data by the AD converter provided in the microcontroller 5. At that time, the sampling period of the AD converter 6 is set automatically by the sampling setting section in the microcontroller 5 according to the above measurement frequency. As described in the above Description of the Related Art, measurement of bioelectrical impedance requires sampling of 20 points per period of measurement signal for the sake of measurement accuracy. That is, the conventional high-speed data processable AD converter requires a sampling period of 1 MHz.

However, as shown in Fig. 4, the AD converter 6 samples one point per period of the above measurement signal, i.e., 20 points in 20 periods of the measurement signal. At that time, the sampling period is set to be longer than the above measurement period by (1 period of signal waveform/number of samples) seconds. More specifically, in the case of the above measurement frequency of 50 kHz, since the measurement period is 20 $\mu$sec, the sampling period is set as 20 $\mu$sec + (20 $\mu$sec/20 points) = 21 $\mu$sec. Thereby, sampling is made with the phase shifted by (360°/20 points) for each sampling with respect to the above measurement signal, resulting in a digital signal waveform as shown in Fig. 4. That is, digital data resulting from sampling 20 points per period of the measurement signal that corresponds to a sampling period of 1 MHz by the above high-speed data processable AD converter are obtained, and the digital data are stored in the storage section in the microcontroller 5.

In STEP S16, as in the above STEP S14, the switching section 13 is switched to the differential amplifier 12 side (referred to as "ch1") to acquire a potential difference that occurs in a living body part between the voltage measuring electrodes 4c and 4d. In STEP S17, digital data is acquired and stored in the storage section in the microcontroller 5, as in the above STEP S15.

Then, in STEP S18, the foregoing DFT process is carried out to calculate an absolute value $|Z_{obj}|$, a phase difference $\phi$ and the resistance component R and reactance component X of bioelectrical impedance which are the above bioelectrical impedance parameters, and the calculated parameters are stored in the storage section in the microcontroller 5.

In subsequent STEP S20, it is determined whether measurements by the preset measurement frequencies have been all completed. In the present example, measurements are made by the two measurement frequencies of 50 kHz and 6.25 kHz. Accordingly, if the measurement by 6.25 kHz has not been made after completion of the measurement by the above measurement frequency of 50 kHz, STEPS S11 to S19 are carried out again as the measurement by the measurement frequency of 6.25 kHz. If the measurements by the two measurement frequencies are completed, the control section returns to the flowchart of the operation of the main routine of Fig. 2.

Example 2

In the above Example 1, one point is sampled per period of the measurement signal with the phase shifted. In Example 2, an example of sampling multiple points per period of measurement signal with the phase shifted will be described by presenting what is different from Example 1.

Firstly, the constitution of the apparatus is the same as that of the body composition measuring apparatus of Example 1 that is shown in Fig. 1. However, the apparatus of Example 2 further comprises a sampling waveform shaping section which shapes data sampled in the AD converter 6 into a predetermined waveform in the microcontroller 5.

Further, the operation of the main routine of Example 2 is the same as that of the main routine of Example 1 that is shown in Fig. 2. The operation of a bioelectrical impedance measurement subroutine is shown in Fig. 5. The subroutine of Fig. 5 is different from the subroutine of Fig. 3 in setting of a sampling period by the sampling setting section of the microcontroller 5 in AD conversion in STEPS S15 and S17 and addition of waveform shaping process by the sampling waveform shaping section in the microcontroller 5 after the above AD conversion. Further, a sampling example when the analog measurement frequency is 5 kHz is shown in Fig. 6 as an example of sampling.

First, according to Fig. 5, when the apparatus reaches measurement of bioelectrical impedance in STEP S5 of the main flowchart of Fig. 2 as in Example 1, the apparatus enters the bioelectrical impedance measurement subroutine of Fig. 5. The operations of STEPS S51 to S54 are the same as those of STEPS S11 to S14 of Fig. 3. In subsequent STEP S55, a potential difference signal in the reference resistance 10 is digitized by the AD converter 6. In this STEP S55, the AD converter 6 samples two points per period of measurement signal, i.e., 20 points in 10 periods of the measurement signal, as shown in Fig. 6. Since desired digital data are obtained with the phase shifted for each sampling with respect to the measurement signal as in Example 1 and two points are sampled per period of the measurement signal, the above sampling period is set to be longer than the half measurement period by (half period of signal waveform/ number of samples) seconds. That is, as indicated by the example of Fig. 6, when the measurement frequency is 5 kHz, the half measurement period is 100 $\mu$sec, and the sampling period is set to be 105 $\mu$sec accordingly. Thereby, sampling is made with the phase shifted by (180°/20 points) for each sampling with respect to the above measurement signal. That is, sampling data resulting from sampling 20 points per period of the measurement signal are obtained and stored in the storage section in the microcontroller 5.

Thus, the apparatus of Example 2 needs only a half of a sampling time of 20 periods (4, 000 $\mu$sec) taken when one point is sampled per period of measurement signal.

In STEP S56, a waveform of one period is shaped from the above sampling data. As shown in Fig. 6, the sampling data are divided into a group of data with odd numbers and a group of data with even numbers based on the sampling numbers of the obtained data, and the data with even numbers are connected to each other and then the data with odd numbers are connected to each other, resulting in a signal of one period. The data are processed in the waveform shaping section in the microcontroller 5. The above stored sampling data are read, shaped and stored in the storage section in the microcontroller 5 sequentially.

In STEP S57, the switching section 13 is switched from ch0 to ch1, as in STEP S16 of Fig. 3. In STEPS S58 and S59, digitization and waveform shaping are performed on a potential difference between living body parts in the same manner as in digitization and waveform shaping of the potential difference signal in the reference resistance in the above STEPS S55 and S56.

The operations of subsequent STEPS S60 and S61 are the same as those of the above STEPS S18 to S20 of Fig. 3.

In Example 2, an example of sampling two points per period of measurement signal has been described. However, it is possible to sample more points per period of the above measurement signal as long as the sampling period does not exceed the processing power of the AD converter 6. Thereby, measurement time can be shortened accordingly.

Further, an example of sampling one point per period of measurement signal has been described in Example 1, and an example of sampling two points per period of measurement signal has been described in Example 2. It is possible to preset use of either sampling pattern according to the processing capability of the AD converter 6 incorporated in the general-purpose microcontroller 5 and measurement frequency or to switch between the sampling patterns based on measurement frequency. For example, it is possible that one point is sampled per period when measurement frequency is 50 kHz (sampling period: 21 $\mu$sec) and two points are sampled per period when measurement frequency is 5 kHz (sampling period: 105 $\mu$sec).

## Claims

1. A bioelectrical impedance measuring device which measures voltages generated in a living body according to alternating currents of predetermined frequencies applied to the living body,
   the device comprising:

   digital data acquiring means, and
   calculation means,
   wherein
   the digital data acquiring means acquires digital data by sampling the measurement signals of the voltages by sampling frequencies that are not higher than the Nyquist frequencies, and
   the calculation means calculates bioelectrical impedance parameter values based on the digital data.

**2.** The device of claim 1, wherein the digital data acquiring means samples the number of samples required to digitize one period of the measurement signal by such a sampling frequency that acquires the number over a number of periods of the measurement signal.

**3.** The device of claim 1 or 2, wherein the digital data acquiring means takes an integer period as the sampling period.

**4.** The device of any one of claims 1 to 3, wherein the digital data acquiring means comprises shaping means for shaping a waveform formed by the sampling when a number of samplings are conducted on one period of the measurement signal.

**5.** The device of any one of claims 1 to 4, wherein the digital data acquiring means comprises sampling frequency switching means for switching the sampling frequency automatically according to the frequency of the measurement signal.

**6.** The device of any one of claims 1 to 5, wherein the calculation means calculates the parameter values by the DFT process based on the digital data.

**7.** A body composition measuring apparatus comprising:

the bioelectrical impedance measuring device of any one of claims 1 to 6, and
body composition calculating means for calculating indicators associated with body compositions such as body fat, muscles, body water and bones based on the acquired parameter values.

# FIG. 1

EEPROM

POWER SOURC

DISPLAY SECTION

AD CONVERTER

BODY WEIGHT MEASURING SECTION

KEY SWITCHES

MICROCONTROLLER

SIGNAL SHAPING FILTERS

SWITCHING SECTION

ALTERNATING CURRENT OUTPUT CIRCUIT

DIFFERENTIAL AMPLIFIER

DIFFERENTIAL AMPLIFIER

REFERENCE RESISTANCE

## FIG. 2

```
        POWER ON
            │
            ▼
   S1                          YES
      PERSONAL ──────────────────┐
    REGISTRATION DONE             │
         ?                        │
            │ NO                  │
            ▼                     │
   S2   COMPLETE PERSONAL         │
         REGISTRATION             │
            │◄────────────────────┘
            ▼
   S3   MAKE INITIAL SETTING
            │
            ▼
   S4   MEASURE BODY WEIGHT
```

```
        ┌──────────────────────────────┐
        ▼                              │
   MEASURE BIOELECTRICAL   ─── S5      │
   IMPEDANCE                           │
            │                          │
            ▼                          │
   CALCULATE BODY          ── S6       │
   COMPOSITIONS                        │
            │◄─────────────────────────┤
            ▼                          │
   DISPLAY RESULTS         ── S7       │
            │                          │
            ▼                 S8       │
      GIVEN PERIOD                  NO │
     OF TIME ELAPSED ────────────────┘
          ?
            │ YES
            ▼
       POWER OFF
```

## FIG. 3

```
   MEASUREMENT OF
   BIOELECTRICAL IMPEDANCE
            │
            ▼
   OUTPUT RECTANGULAR WAVE      ── S11
            │
            ▼
   SELECT SHAPING FILTER OF     ── S12
   PREDETERMINED FREQUENCY
            │
            ▼
   APPLY CURRENT TO REFERENCE   ── S13
   RESISTANCE AND BODY
            │
            ▼
   SWITCH SWITCHING SECTION     ── S14
   TO SELECT ch0
            │
            ▼
   DIGITIZE POTENTIAL           ── S15
   DIFFERENCE SIGNAL FROM
   REFERENCE RESISTANCE
            │
            ▼
   SWITCH SWITCHING SECTION     ── S16
   TO SELECT ch1
            │
            └──────────┐
```

```
        ┌──────────────────────────────┐
        ▼                              │
   DIGITIZE POTENTIAL DIFFERENCE ── S17 │
   SIGNAL FROM LIVING BODY            │
            │                          │
            ▼                          │
   PERFORM DFT PROCESS         ── S18  │
            │                          │
            ▼                          │
   CALCULATE IMPEDANCE         ── S19  │
   PARAMETERS                          │
            │                          │
            ▼                 S20      │
      ALL MEASUREMENTS              NO │
   BY PRESET MEASUREMENT ─────────────┘
   FREQUENCIES COMPLETED
          ?
            │ YES
            ▼
        RETURN
```

# FIG. 4

ANALOG SIGNAL : 50kHz (PERIOD 20 $\mu$ sec)

DIGITAL SIGNAL WAVEFORM

SAMPLING PERIOD 21 $\mu$ sec

# FIG. 5

MEASUREMENT OF BIOELECTRICAL IMPEDANCE

OUTPUT RECTANGULAR WAVE — S51

SELECT SHAPING FILTER OF PREDETERMINED FREQUENCY — S52

APPLY CURRENT TO REFERENCE RESISTANCE AND BODY — S53

SWITCH SWITCHING SECTION TO SELECT ch0 — S54

DIGITIZE POTENTIAL DIFFERENCE SIGNAL FROM REFERENCE RESISTANCE — S55

SHAPE SAMPLING WAVEFORM — S56

SWITCH SWITCHING SECTION TO SELECT ch1 — S57

DIGITIZE POTENTIAL DIFFERENCE SIGNAL FROM LIVING BODY — S58

SHAPE SAMPLING WAVEFORM — S59

PERFORM DFT PROCESS — S60

CALCULATE IMPEDANCE PARAMETERS — S61

ALL MEASUREMENTS BY PRESET MEASUREMENT FREQUENCIES COMPLETED ? — S62

NO

YES

RETURN

# FIG. 6

ANALOG SIGNAL : 5kHz
(PERIOD 200 $\mu$ sec)

DIGITAL SIGNAL WAVEFORM

SAMPLING PERIOD 105 $\mu$ sec

# FIG. 7

100 CURRENT SOURCE

103 DIFFERENTIAL AMPLIFIER

CURRENT i

101 REFERENCE RESISTANCE (Ref)

107 DFT

106 HIGH-SPEED AD CONVETER

105 SW

102 LIVING BODY (Obj)

104 DIFFERENTIAL AMPLIFIER

**European Patent Office**

# EUROPEAN SEARCH REPORT

Application Number

EP 06 00 1348

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/158167 A1 (SMITH KENNETH CARLESS ET AL) 12 August 2004 (2004-08-12) | 1-5 | INV. A61B5/053 |
| Y | * paragraphs [0076], [0080], [0087], [0090], [0092], [0093], [0095] * ----- | 6,7 | |
| Y | WO 2004/047635 A (IMPEDIMED PTY LTD; KENNEDY, JAMES) 10 June 2004 (2004-06-10) * column 1, lines 12-24 * * column 10, lines 9-22 * ----- | 6,7 | |
| P,X | WO 2005/122889 A (TALLINN UNIVERSITY OF TECHNOLOGY; MIN, MART; LAND, RAUL; PARVE, TOOMAS) 29 December 2005 (2005-12-29) * column 5, line 32 - column 9, line 19 * ----- | 1-4 | |
| | | | TECHNICAL FIELDS SEARCHED (IPC) A61B |

The present search report has been drawn up for all claims

| Place of search | Date of completion of the search | Examiner |
|---|---|---|
| Munich | 7 April 2006 | Martelli, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT**
**ON EUROPEAN PATENT APPLICATION NO.**

EP 06 00 1348

This annex lists the patent family members relating to the patent documents cited in the above-mentioned European search report.
The members are as contained in the European Patent Office EDP file on
The European Patent Office is in no way liable for these particulars which are merely given for the purpose of information.

07-04-2006

| Patent document cited in search report | | Publication date | Patent family member(s) | | Publication date |
|---|---|---|---|---|---|
| US 2004158167 | A1 | 12-08-2004 | NONE | | |
| WO 2004047635 | A | 10-06-2004 | EP | 1565105 A1 | 24-08-2005 |
| | | | JP | 2006507057 T | 02-03-2006 |
| WO 2005122889 | A | 29-12-2005 | NONE | | |

EPO FORM P0459